# EUROPEAN PATENT SPECIFICATION

(11) **EP 3 709 887 B1**
(45) Date of publication and mention of the grant of the patent: **27.09.2023**
(21) Application number: 18807444.7
(22) Date of filing: 09.11.2018
(51) Int. Cl.: A61B 8/00

(54) **ULTRASONIC PROBE AND ULTRASONIC MEASUREMENT SYSTEM**
ULTRASCHALLSONDE UND ULTRASCHALLMESSSYSTEM
SONDE À ULTRASONS ET SYSTÈME DE MESURE PAR ULTRASONS

(30) Priority: 17.11.2017 JP 2017221591
(43) Date of publication of application: 23.09.2020
(73) Proprietor: Nihon Kohden Corporation, Tokyo 161-8560 (JP)
(72) Inventor: OURA, Mitsuhiro, Tokorozawa-shi Saitama 359-0037 (JP); KUMAGAI, Sou, Tokorozawa-shi Saitama 359-0037 (JP); MATSUZAWA, Wataru, Tokorozawa-shi Saitama 359-0037 (JP); YASUMARU, Nobuyuki, Tokorozawa-shi Saitama 359-0037 (JP); NAGASE, Kazuya, Tokorozawa-shi Saitama 359-0037 (JP); TORIGAI, Hiroshi, Tokyo 161-8560 (JP); FUKUSHIMA, Naoki, Tokyo 161-8560 (JP); SATO, Masashi, Tokorozawa-shi Saitama 359-0037 (JP); AIZAWA, Takuya, Tokorozawa-shi Saitama 359-0037 (JP)
(74) Representative: Grünecker Patent- und Rechtsanwälte PartG mbB
(86) International application number: PCT/JP2018/041628
(87) International publication number: WO 2019/098132

(56) References cited:
- WO-A1-2017/135769
- KR-B1- 101 792 952
- US-A1- 2006 058 654
- US-A1- 2007 167 709
- US-A1- 2013 023 741
- US-A1- 2013 237 811
- Offiah: "Imaging assessment of penetrating craniocerebral and spinal trauma", Clinical Radiology, 1 January 2009 (2009-01-01), pages 1146-1157, XP055557239, England DOI: 10.1016/j.crad.2009.06.004 Retrieved from the Internet: URL:https://static1.squarespace.com/static /558625f9e4b0363097775e40/t/55899b99e4b000 62e986a1f8/1435081625699/Clear+Guide+ONE-B rochure.20150306.compr_.pdf [retrieved on 2019-02-14]

## Description

### Technical Field

The presently disclosed subject matter relates to an ultrasonic probe and an ultrasonic measurement system.

### Background Art

Ultrasonic diagnostic apparatuses are widely used for checking a condition of a chest, an abdomen, etc. of a subject. The ultrasonic diagnostic apparatuses configured to cause an ultrasonic beam enter a body of the subject through a probe head of an ultrasonic probe, and processes a reflected wave of the ultrasonic beam to thereby display an ultrasonic image (e.g., a tomographic image inside the body or a blood flow image). When displaying the image, an abutment position, an abutment angle etc. of the probe head are recorded. When this record is absent, the abutment position, the abutment angle, etc. of the probe head may be then unclear to thereby make diagnosis difficult.

To solve this problem, in a general ultrasonic diagnostic apparatus, a body mark (a kind of icon) is displayed together with an ultrasonic image on a screen. The body mark is a schematic diagram illustrating the abutment position or an abutment direction of the probe head. The body mark is stored in association with the ultrasonic image. The body mark is usually input or set manually by an operator before or after imaging.

However, when the operator changes the abutment position or the abutment angle, the operator has to input the body mark. Such an operation burden is large. In addition, there is also a problem that it is difficult to know which direction the image was captured from, based on information of only the body mark.

An ultrasonic diagnostic apparatus according to a first related art (see, e.g., JP2006-000400A) has a video camera taking an image of a subject and an ultrasonic probe and displays an image in which an ultrasonic image and the image of the video camera have been combined. Thus, it is possible to grasp information about an abutment region or an abutment angle without performing an input work for a body mark.

A second related art (see, e.g., JP2005-058577A) also has a configuration similar to the first related art, i.e., a configuration in which an imaging device is provided to capture an image of an ultrasonic probe.

According to a third related art, an ultrasonic image is displayed together with various vital signs on a patient monitor (see, e.g., WO2009/138902A1). In the configuration, the patient monitor is configured to be detachably attached to an ultrasonic probe. The patient monitor displays the ultrasonic image together with information of the vital signs (e.g., blood pressure, a pulse, a respiration rate, body temperature, etc.). In the configuration, the ultrasonic probe is inserted into a connector insertion port of the patient monitor so that ultrasonic diagnosis can be made simply (see, e.g., Fig. 1 of WO2009/138902A1).

However, in the third related art, there is no suggestion or instruction about a body mark. Accordingly, a manual input burden is large when the body mark is used.

The first related art and the second related art have a configuration in which the imaging device (the apparatus imaging the ultrasonic probe) is provided to have a separate housing from that of the ultrasonic diagnostic apparatus (see, e.g., Fig. 1 of JP2006-000400A and Fig. 1 of JP2005-058577A). That is, the ultrasonic probe, the apparatus (the ultrasonic diagnostic apparatus) processing a signal acquired from the ultrasonic probe and displaying the processed signal, and the imaging device are provided separately. However, since the patient monitor is assumed to be used at various scenes, it may be difficult to provide the imaging device having the separate housing.

For example, in a case where an ultrasonic image is desired to be referred to with the patient monitor being used in an operating room, the degree of spatial freedom is significantly impaired if a body mark imaging device is also provided in the operating room having a large physical limitation. Even when the ultrasonic probe is desired to be connected to the patient monitor only if the occasion demands, the imaging device has to be additionally provided.

The first related art and the second related art assume that the imaging device is fixed at a place to capture a photographic image. Accordingly, an image of the probe head may not be captured properly and an abutment state of the probe head may not be grasped accurately.

This problem (the impairment of the degree of spatial freedom due to the provision of the imaging device) is not limited to the case where the patient monitor supporting the ultrasonic image is used in the operating room, but is a problem common to a case where the ultrasonic probe is used in connection with the patient monitor. Also in a case where the ultrasonic probe is connected to a tablet type personal computer, there is a need that an abutment state of the probe head is desired to be grasped. The impairment of the degree of spatial freedom due to the arrangement of the imaging device at a fixed point is desirably avoided also in the case where the tablet type personal computer is used.

Document US 2007/167709 discloses an ultrasonic probe according to the preamble of claim 1.

### Summary of Invention

The invention is defined in the independent claims.
Illustrative aspects of the presently disclosed subject matter provide an ultrasonic probe and an ultrasonic measurement system in which an abutment state of a probe part (probe head) of the ultrasonic probe can be grasped properly in a simple configuration when the ultrasonic probe is used in connection with an apparatus displaying an ultrasonic image.

According to an aspect of the presently disclosed subject matter, an ultrasonic probe according to claim 1 is provided.

With this configuration, the image capturing unit is configured to capture an image of the nearby object, and is connectable to the probe head through the first cable. Thus, the image capturing unit can capture an image of the probe head from a distant position. A user can hold and move the image capturing unit to capture an image of an abutment state of the probe head. In addition, the image capturing unit and the probe head are integrated with each other through the first cable. Since the image capturing unit and the probe head are integrated with each other, it is possible to capture an image of a condition of a nearby object during ultrasonic diagnosis without providing a separate imaging device. That is, even at a place with a large physical limitation, it is possible to perform ultrasonic measurement while grasping the abutment state of the ultrasonic probe in a simple configuration.

### Brief Description of Drawings

[fig.1]Fig. 1 is a view of an ultrasonic probe and a patient monitor according to an embodiment of the presently disclosed subject matter.
[fig.2]Fig. 2 is another view of the ultrasonic probe.
[fig.3A]Fig. 3A is a view of an image capturing unit of the ultrasonic probe.
[fig.3B]Fig. 3B is another view of the image capturing unit.
[fig.4A]Fig. 4A is a view of a probe head of the ultrasonic probe.
[fig.4B]Fig. 4B is another view of the probe head.
[fig.5]Fig. 5 is a view illustrating an example of how the ultrasonic probe is to be used.
[fig.6]Fig. 6 is a view of another example of the ultrasonic probe.
[fig.7A]Fig. 7A is a view of another example of the image capturing unit.
[fig.7B]Fig. 7B is another view of the image capturing unit of Fig. 7A.
[fig.8]Fig. 8 is a view illustrating another example of how the ultrasonic probe is to be used.

### Description of Embodiments

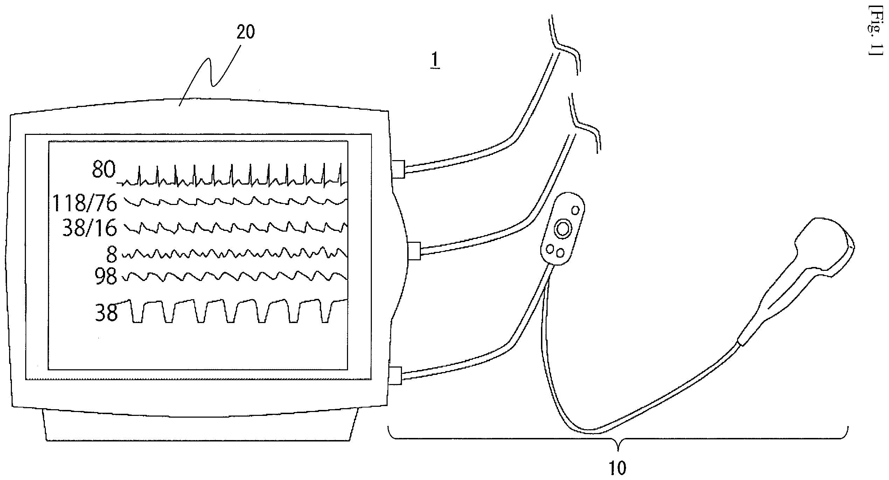

Embodiments of the presently disclosed subject matter will be described below with reference to the drawings. Fig. 1 is a view of an ultrasonic measurement system 1. The ultrasonic measurement system 1 includes an ultrasonic probe 10 and a patient monitor 20 connected to the ultrasonic probe 10. The patient monitor 20 is configured to measure various vital signs based on vital sign signals acquired by various sensors (not illustrated in Fig. 1). The sensors may include, for example, a cuff used for measurement of blood pressure, electrodes (such as disposable electrodes, clip electrodes, etc.) used for measurement of an electrocardiogram etc., an SpO2 probe, a mask used for measurement of respiration, etc. The vital signs may include, for example, body temperature, the blood pressure, the electrocardiogram, respiration information (a respiration rate, a respiration waveform), SpO2 (arterial oxygen saturation), a heart rate, etc. The number of the vital signs to be measured by the patient monitor 20 or the number of the sensors to be connected to the patient monitor 20 may be set desirably. The patient monitor 20 is a concept that can be interpreted to correspond to various medical apparatuses for measuring the various vital signs, such as a bedside monitor, a wearable medical telemeter, a defibrillator including a measurement function of the electrocardiogram etc., etc. In the following description, assume that the patient monitor 20 is a so-called bedside monitor.

The patient monitor 20 can be electrically connected to (can transmit/receive an electronic signal to/from) the ultrasonic probe 10 in addition to the aforementioned various sensors. When a connector of the ultrasonic probe 10 is inserted into a connector insertion port of the patient monitor 20 in the configuration of Fig. 1, the ultrasonic probe 10 is electrically connected to the patient monitor 20. The ultrasonic probe 10 abuts on a body surface of a subject, transmits an ultrasonic beam toward the body surface, and receives a signal representing a reflected wave from the body surface. The ultrasonic probe 10 supplies the reflected wave signal to the patient monitor 20. The ultrasonic probe 10 may supply the reflected wave signal directly to the patient monitor 20, or may perform various signal processings on the reflected wave signal and supply ultrasonic image data created thus to the patient monitor 20. That is, the ultrasonic probe 10 supplies the image information about the ultrasonic wave to the patient monitor 20.

The patient monitor 20 has a display that displays measured values and measured waveforms of the various vital signs, as illustrated in Fig. 1. The waveforms of the vital signs in the patient monitor 20 may be analyzed or displayed by a generally used method. In addition, the patient monitor 20 displays an ultrasonic image of the subject on the display based on the reflected wave signal (or the ultrasonic image data) received from the ultrasonic probe 10. That is, the patient monitor 20 has a configuration in which ultrasonic image displaying software can be installed into the patient monitor 20. Specifically, the patient monitor 20 includes various storage devices (a hard disk, an RAM, an ROM, etc.) configured to store the software, a central processing unit (CPU) configured to read and execute data (including the software) from the storage devices, etc.

The patient monitor 20 may display the ultrasonic image together with the measured values or the measured waveforms of the vital signs or may display only the ultrasonic image. In addition, the patient monitor 20 displays a photographic image (a still image or a moving image) captured by an image capturing unit 12 on the display. The image capturing unit 12 will be described later. The patient monitor 20 properly records the ultrasonic image or the photographic image on an internal recording device (e.g., the hard disk).

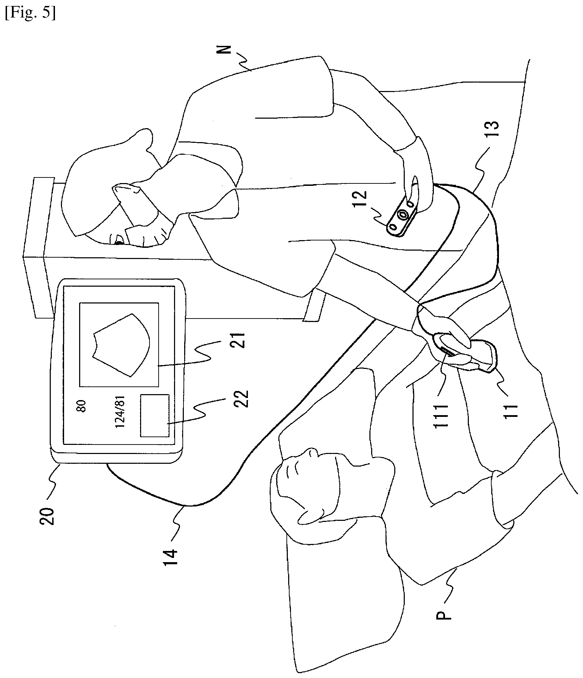

The patient monitor 20 may detect characteristics (an output frequency, output intensity, a probe type, etc.) of the ultrasonic probe 10 based on at least one of a color and a shape of a marker 111 (that will be described later) of a probe head 11. The detection will be described later with reference to Fig. 5.

When a control signal for instructing a change of settings (e.g., alarm cancellation, activation of one of various applications, etc.) is input to the patient monitor 20 from the ultrasonic probe 10, the patient monitor 20 changes the setting of the patient monitor 20 itself or activates the application in accordance with the control signal. An electric circuit and/or a program for processing the input of the control signal may be provided in the patient monitor 20.

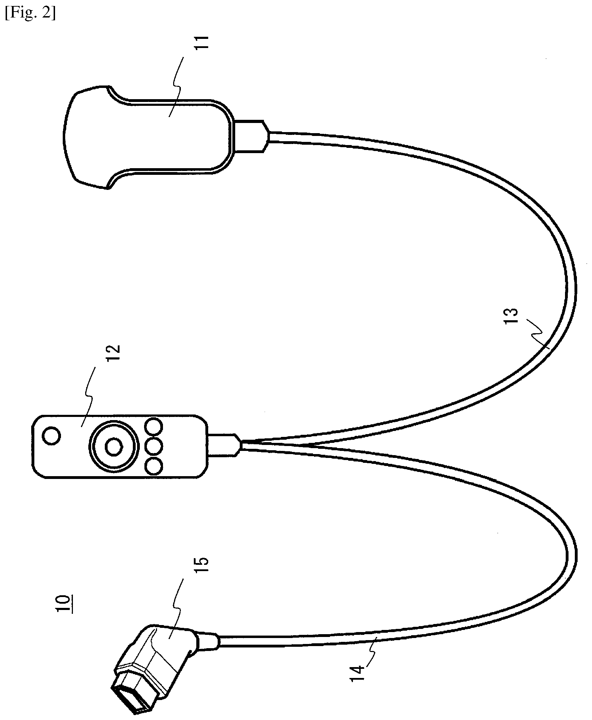

Next, a housing configuration of the ultrasonic probe 10 will be described with reference to Fig. 2. The ultrasonic probe 10 has the probe head 11, the image capturing unit 12, a cable 13 (first cable), a cable 14 (second cable), and a connector 15. The cable 13 and the cable 14 may have optional lengths respectively. The cable 13 and the cable 14 can be bent flexibly.

The probe head 11 abuts on a body surface of a subject (i.e. makes contact with the body surface of the subject or is sufficiently close to the body surface of the subject), transmits an ultrasonic beam on the body surface of the subject, and receives a reflected wave of the ultrasonic beam from the body surface of the subject. The probe head 11 may be of a sector type, a convex type, a linear type or any other type. The probe head 11 is installed with various electric circuits and the like to control an ultrasonic frequency, beam forming, mode switch (e.g., switching among a B mode, an M mode and a D mode), contrast, depth, an imaging gain, etc.. The probe head 11 supplies an image signal based on the reflected wave to the patient monitor 20 through the cable 13 and the cable 14.

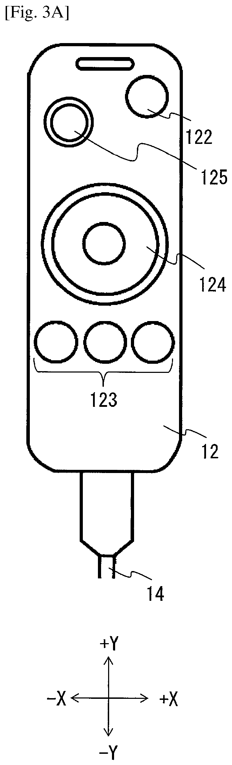

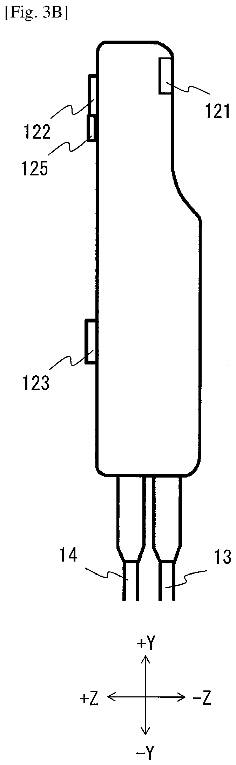

The configuration of the image capturing unit 12 will be described with reference to Figs. 3A and 3B in addition to Fig. 2. Fig. 3A is a conceptual diagram in which an operating face of the image capturing unit 12 is viewed as a front face (the face where input interfaces that will be described later are provided is viewed as the front face). Fig. 3B is a conceptual diagram in which the image capturing unit 12 is viewed from a side. Description will be made on the assumption that a short axis direction, a long axis direction and a depth direction in the front view and the side view of the image capturing unit 12 in Figs. 3A and 3B are expressed as X-axis direction, Y-axis direction and Z-axis direction respectively. A user holds the image capturing unit 12 so as to pinch the image capturing unit 12 from a +Z direction and a -Z direction though not always limited thereto. More specifically, the user presses the image capturing unit 12 by a thumb from the +Z direction and presses the image capturing unit 12 by fingers other than the thumb from the -Z direction.

The image capturing unit 12 has an optical lens 121 (first optical lens in Fig. 3B) for capturing an image of a nearby object. A position where the optical lens 121 is disposed is not limited particularly, but is located on an upper portion side of a long-axis face disposed in the -Z direction (a +Y side, in other words, a side far from a face to which the cable 13 and the cable 14 are connected when the long axis (the length in the Y-axis direction) of the image capturing unit 12 is divided into two). The optical lens 121 may be an optical lens suitable for taking a moving image or a still image, or an optical lens similar to or the same as that provided, for example, in a smartphone etc.

The various input interfaces are provided on a housing of the image capturing unit 12. In the example of Figs. 3A and 3B, a power button 122, input buttons 123, a scroll wheel 124 are provided on the long-axis face in the +Z direction. The power button 122 is a button for controlling ON/OFF of the image capturing unit 12. The input buttons 123 are used for instructing start or stop of imaging. The scroll wheel 124 is also used for instructing start or stop of imaging in a similar manner to or the same manner as the input buttons.

The input interfaces in Figs. 3A and 3B are merely examples. The number of the buttons or the positions where the buttons are disposed are not particularly limited. The input interfaces (the power button 122, the input buttons 123 and the scroll wheel 124) may be provided on a side opposite to the side on which the optical lens 121 is provided, as illustrated in Fig. 3B. With this configuration, it is possible to stop imaging or issue an instruction etc. to the probe head 11 that will be described later even during the imaging without touching the optical lens 121 (in other words, without interrupting the imaging). The image capturing unit 12 may have a configuration in which some of the input interfaces are provided on one or each of the side faces.

Although not essential, the image capturing unit 12 may be configured to have the optical lens 121 and other optical lenses. In the configuration in Fig. 3A, an optical lens 125 is provided on the long-axis face in the +Z direction. Thus, it is possible to image not only an abutment state of the probe head 11 but also information of a person who is engaging in the imaging, etc.

The image capturing unit 12 has an internal configuration equivalent to a general digital still camera. For example, the image capturing unit 12 has input/output interfaces, various circuits (an analog signal processing circuit, an A/D converter, a digital signal processing circuit, an image inputting controller, etc.), various storage devices (a random access memory (RAM), a read only memory (ROM), etc.), a central processing unit (CPU) or a micro processing unit (MPU), a diaphragm, a diaphragm actuator, etc. The image capturing unit 12 applies photoelectric conversion etc. to light that has passed through the optical lens 121 and the diaphragm to thereby create an image signal (a signal indicating a photographic image). The image capturing unit 12 may be configured to perform various image processings (e.g., white balance processing) etc. The image capturing unit 12 transmits the image signal to the patient monitor 20 through the cable 14.

The image capturing unit 12 may act as a remote controller that transmits a control signal to at least one of the patient monitor 20 and the probe head 11. For example, the image capturing unit 12 may transmit, to the probe head 11, a control signal for instructing change of the ultrasonic frequency, change of the beam forming setting, the mode switch (e.g., among the B mode, the M mode and the D mode), the contrast, the depth, the imaging gain, etc. in accordance with operation on the input interfaces (the input buttons 123 or the scroll wheel 124). The image capturing unit 12 may transmit, to the patient monitor 20, a control signal for instructing alarm cancellation, display setting, activation of an application etc. in accordance with operation on the input interfaces. That is, the image capturing unit 12 is configured to transmit a control signal for instructing a change of settings to at least one of the patient monitor 20 and the probe head 11 in accordance with an operation on the input interfaces provided on the housing. Upon receipt of the control signal as the input, the probe head 11 changes the settings in accordance with the control signal. To transmit the control signal to the probe head 11, the image capturing unit 12 may transmit the control signal via the patient monitor 20.

The image capturing unit 12 is connected to the cable 13 and the cable 14 (Fig. 3A). The cable 13 may be joined to the image capturing unit 12 (in other words, the cable 13 may be fixed to the image capturing unit 12 such that the cable 13 cannot be inserted into or removed out of the image capturing unit 12). Alternatively, the image capturing unit 12 and the cable 13 may be configured to be detachably attached to each other. That is, the image capturing unit 12 is joined to or detachably attached to the cable 13. The image capturing unit 12 has the optical lens 121 to capture an image of a nearby object.

In a similar manner or the same manner, the cable 14 may be joined to the image capturing unit 12, or the image capturing unit 12 and the cable 14 may be configured to be detachably attached to each other.

It is desirable that a connection place of the cable 13 and a connection place of the cable 14 are located in the same face, as illustrated in Fig. 3B. In addition, it is preferable that the optical lens 121 is disposed on the face different from (another face than) the face where the cable 13 and the cable 14 are connected. In the example of Fig. 3B, the cable 13 and the cable 14 extend from the side facing in a -Y direction. That is, an extension direction of the cable 13 and an extension direction of the cable 14 are substantially the same, and the cable 13 and the cable 14 extend in the direction (the -Y direction) that is different from an imaging direction (the -Z direction) of the optical lens 121. The cable 13 and the cable 14 are connected to the image capturing unit 12 at positions separated from the optical lens 121. The user typically holds the image capturing unit 12 such that the optical lens 121 is on an upper side (an anti-gravitational side). Therefore, when the user holding the image capturing unit 12 captures a photographic image, the cable 13 and the cable 14 extend in the gravity direction so that the cable 13 and the cable 14 can be prevented from easily entering the imaging range of the optical lens 121. That is, the cable 13 and the cable 14 are connected to the face (the side facing in the -Y direction) different from the face (the side facing in the -Z direction) where the optical lens 121 is disposed. Thus, reflection of the cable 13 and the cable 14 on the photographic image can be prevented. The face (the side facing in the -Y direction) where the connection place of the cable 13 and the connection place of the cable 14 are disposed does not have to be a planar face but may be a slightly round curved face alternatively.

A relation between the cables 13 14 and the optical lens 121 will be further described. When the optical lens 121 is oriented in a substantially horizontal direction (the -Z direction), i.e., in a direction substantially parallel to a ground surface, the cable 13 and the cable 14 extends substantially in the gravity direction (the -Y direction). Thus, an angle formed between the imaging direction of the optical lens 121 and the extension direction of the cable 13 and the cable 14 is sufficiently large, and the extension direction of the cable 13 and the cable 14 is substantially the gravity direction. Accordingly, the cable 13 and the cable 14 can be prevented from being captured in an image during imaging.

When the optical lens 121 is viewed from the front (when the image capturing unit 12 is viewed from the -Z direction), the cable 13 and the cable 14 are connected to the image capturing unit 12 such that the cable 13 and the cable 14 are arranged one behind the other in the depth direction (in the Z-axis direction) to be. Since the cable 13 and the cable 14 extend from the image capturing unit 12 such that they are arranged one behind the other in the depth direction, the cable 13 and the cable 14 can be prevented from being obstacles to operation (in other words, easy to handle) when the user holds the image capturing unit 12.

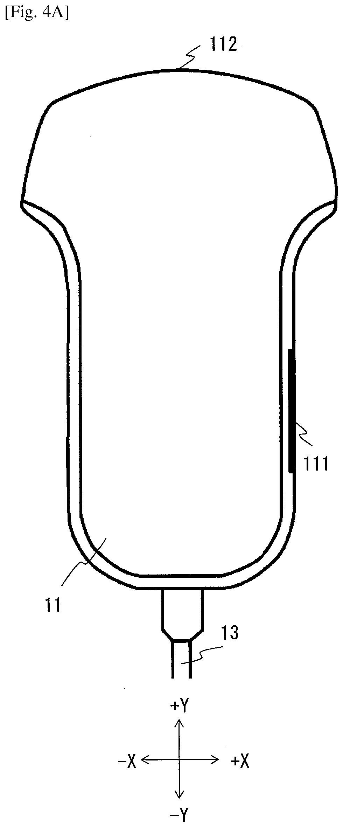

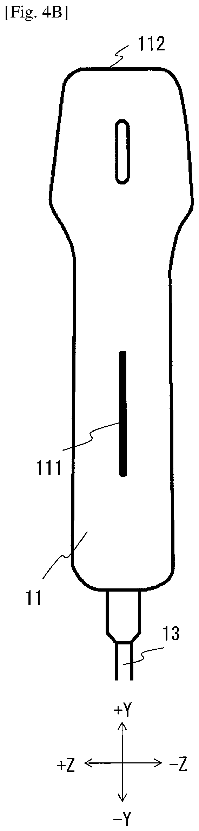

Next, the configuration of the probe head 11 will be described with reference to Fig. 2 and Figs. 4A and 4B. Fig. 4A is a front view of the probe head 11. Fig. 4B is a side view of the probe head 11. In Figs. 4A and 4B, a short axis direction, a long axis direction and a depth direction of the probe head 11 represent the X-axis direction, the Y-axis direction and the Z-axis direction respectively. The user typically holds the probe head 11 so as to pinch the probe head 11 from the -Z direction and the +Z direction.

The probe head 11 may have a shape or a configuration similar to or the same as that of a probe head used for ordinary ultrasonic measurement. It is preferable that the probe head 11 has the marker 111 according to which the left and the right can be grasped, as illustrated in Figs. 4A and 4B. Any marker can be used as the marker 111 as long as the left and right of the probe head 11 can be identified according to the marker. The shape of the marker 111 and the number of markers 111 may be set desirably. Any marker can be used as the marker 111 as long as an upper portion and a lower portion of the probe head 11 can be grasped according to the marker. That is, any marker can be used as the marker 111 as long as an abutment direction of the probe head 11 on the body surface of the subject can be recognized according to the marker. When a photographic image of the probe head 11 captured by the image capturing unit 12 is referred to, the user refers to the position of the marker 111 inside the photographic image. The user can more accurately determine how the probe head 11 has abutted on the body surface of the subject according to the reference position of the marker 111.

The marker 111 may have the color or shape corresponding to the characteristics (for example, the output frequency, the output intensity, the probe type such as convex, linear, sector, etc.) of the ultrasonic probe 10. For example, when the frequency that can be output is lower than 2.5 MHz, the marker 111 may be blue. When the frequency that can be output is not lower than 2.5 MHz, the marker 111 may be red.

The probe head 11 has an abutment face 112 abutting on the subject. The abutment face 112 abuts on the body surface of the subject and transmits an ultrasonic beam thereon. The abutment face 112 receives a reflected wave from the body surface of the subject. The probe head 11 supplies a signal of the reflected wave to the patient monitor 20 through the cable 13 and the cable 14. The probe head 11 may apply signal processing to the reflected wave signal to thereby create ultrasonic image data, and transmit the ultrasonic image data to the patient monitor 20. The probe head 11 includes various electric circuits inside the probe head 11 to transmit the ultrasonic beam and also to receive the reflected wave of the ultrasonic beam.

The cable 13 is a flexible cable through which the image capturing unit 12 and the probe head 11 are connected to each other. The cable 13 transmits an electric signal between the image capturing unit 12 and the probe head 11.

The cable 14 is a flexible cable through which the image capturing unit 12 and the patient monitor 20 are connected to each other. The cable 14 transfers an electric signal between the image capturing unit 12 and the patient monitor 20. The other end of the cable 14 is connected to the connector 15. The connector 15 is inserted into a vacant slot (connection port) of the patient monitor 20 to be thereby connected to the patient monitor 20. It is desirable that the connector 15 has a universal shape that can be inserted into a general patient monitor 20. The cable 14 is connected to the connector 15, and joined to or detachably attached to the image capturing unit 12.

Next, an example of how the ultrasonic probe 10 is to be used will be described with reference to Fig. 5. Fig. 5 is a conceptual diagram illustrating a use form in which the ultrasonic probe 10 is connected to a wall hanging type patient monitor 20. The configuration of Fig. 5 is merely an example. It is a matter of course that the patient monitor 20 may be of any other type than the wall hanging type.

A nurse N holds the image capturing unit 12 by her/his left hand, and holds the probe head 11 by her/his right hand. The image capturing unit 12 is connected to the patient monitor 20 through the cable 14. The probe head 11 is connected to the image capturing unit 12 through the cable 13.

The nurse N places the probe head 11 in contact with a body surface in the vicinity of an abdomen of a subject P to thereby perform ultrasonic measurement thereon. At the same time, after validating (turning ON) an imaging function of the image capturing unit 12, the nurse N captures a photographic image by the optical lens 121 (not illustrated in Fig. 5) oriented toward the probe head 11. That is, the image capturing unit 12 is configured to be able to capture a photographic image (may be a moving image or may be a still image) of a nearby object. Preferably, the image capturing unit 12 capture a photographic image of an abutment state of the probe head 11.

A reflected wave signal acquired by the probe head 11 is supplied to the patient monitor 20 through the cable 13 and the cable 14. Ultrasonic image data may be alternatively supplied to the patient monitor 20 after the reflected wave signal has been converted into the ultrasonic image data. The image capturing unit 12 supplies the photographic image (the moving image or the still image) to the patient monitor 20 through the cable 14.

The patient monitor 20 displays an ultrasonic image 21 (an abdominal echo image in this example) of the subject P on the display based on the supplied reflected wave signal (or ultrasonic image data). The patient monitor 20 also displays, on the display, the photographic image 22 (the moving image or the still image) acquired by the image capturing unit 12 in place of a body mark. The display example in Fig. 5 is merely an example, and the display form may be set desirably. The nurse N may optionally select a preferred mode to display only the ultrasonic image 21 or to display only the photographic image 22. The patient monitor 20 may also display measured values (e.g., blood pressure values) or measured waveforms of vital signs acquired by the not-shown sensors. The photographic image 22 is treated equivalently to a body mark image used in a general ultrasonic measurement apparatus. The nurse N does not have to perform an operation of inputting the body mark but can refer to the photographic image 22 equivalent to the body mark only by pointing the optical lens 121 of the image capturing unit 12 at a direction where the probe head 11 is present.

It is desirable that the patient monitor 20 displays the ultrasonic image 21 and the photographic image 22 and records them on the built-in hard disk etc. in association with time information. For example, the patient monitor 20 may record the ultrasonic image 21 or the photographic image 22 in association with the time information in a form of a digital imaging and communication in medicine (DICOM).

The nurse N may operate the input interfaces (the buttons etc.) of the image capturing unit 12 to issue an instruction for mode change about an ultrasonic wave, adjustment of the image quality, etc. The nurse N grasps the condition with reference to the ultrasonic image 21, and operates the image capturing unit 12 (operates the input interfaces) to adjust the gain, depth, contrast, frequency etc. during the ultrasonic measurement if necessary. The image capturing unit 12 transmits a control signal for instructing a change of settings (change of the depth or frequency) to the probe head 11. In a similar manner or the same manner, the nurse N may operate the image capturing unit 12 (operate the input interfaces) to perform the setting change (e.g., alarm cancellation) of the patient monitor 20. The image capturing unit 12 transmits the control signal to the patient monitor 20 in accordance with an input instruction for the alarm cancellation etc. By referring to a large screen of the patient monitor 20, the nurse N can accurately grasp the ultrasonic image 21 or the photographic image 22 and change the setting of the probe head 11 or the patient monitor 20 based on the accurately grasped information. In addition, the nurse N operates the image capturing unit 12 on hand to change the setting. Accordingly, while continuing to capture a photographic image, the nurse N can change the setting of the probe head 11 or the patient monitor 20 without taking eyes off the display of the patient monitor 20. The setting change of the optical lens 121 may be performed properly in accordance with operation on the input interfaces.

It is preferable that the image capturing unit 12 is configured to be able to transmit the control signal to each of the patient monitor 20 and the probe head 11. However, the image capturing unit 12 may be alternatively configured to transmit the control signal to one of the patient monitor 20 and the probe head 11.

Next, actions of the vital signal information monitor 20 when the marker 111 has the color or the shape corresponding to the characteristics of the ultrasonic probe 10 will be described. The patient monitor 20 may analyze at least one of the color and the shape of the marker 111 reflected inside the photographic image 22, and detect the characteristics of the ultrasonic probe 10 (the output frequency, the output intensity, the probe type (convex, linear, sector, etc.), etc.). The patient monitor 20 may store a table etc. showing the relation between the color or shape of the marker 111 and the characteristics of the ultrasonic probe 10 in advance so that the patient monitor 20 can detect the characteristics of the ultrasonic probe 10 based on the information obtained from the photographic image 22 based on the image analysis and the table. The patient monitor 20 may display the detected characteristics of the ultrasonic probe 10 on the display or write the detected characteristics of the ultrasonic probe 10 on the internal data storage device (e.g., the hard disk).

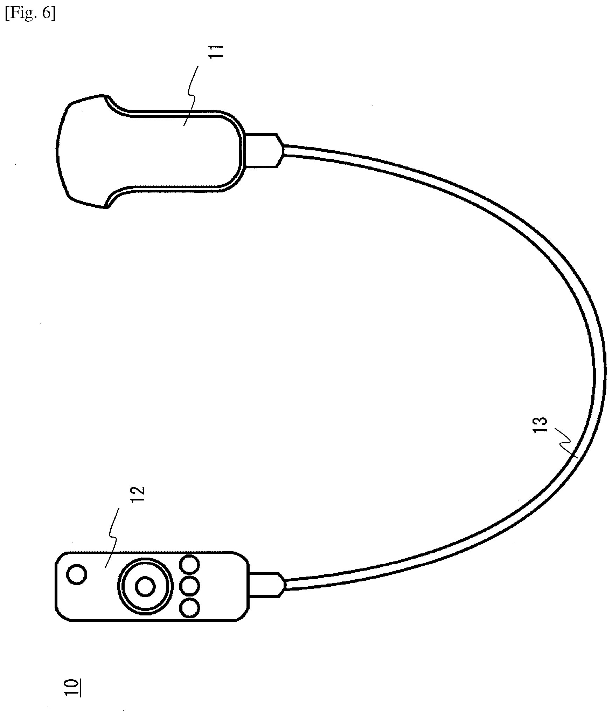

The configuration of the ultrasonic probe 10 is not limited to the one illustrated in Fig. 2. The ultrasonic probe 10 may be alternatively configured in such a manner that no cable 14 extends from the image capturing unit 12, as illustrated in Fig. 6. That is, the image capturing unit 12 and the patient monitor 20 may transmit and receive data by a wireless connection function (e.g., a short range wireless communication function). The ultrasonic probe 10 may be configured to be able to transmit/receive an electric signal to/from (configured to be able to be electrically connected to) the patient monitor 20, or may be connected to the patient monitor 20 by wire or by wireless. In a similar manner or the same manner, the probe head 11 and the patient monitor 20 may transmit and receive data by the wireless connection function (e.g., the short range wireless communication function). The probe head 11 may transmit data to the image capturing unit 12 by wire, and only the image capturing unit 12 may transmit and receive data to and from the patient monitor 20 by wireless. The ultrasonic probe 10 illustrated in Fig. 6 has a configuration in which the image capturing unit 12 and the probe head 11 are connected to each other through the cable 13. The cable 13 and the image capturing unit 12 may be joined to or detachably attached to each other. In a similar manner or the same manner, the cable 13 and the probe head 11 may be joined to or detachably attached to each other. The configuration of the image capturing unit 12 may be similar to or the same as that illustrated in Figs. 3A and 3B. The configuration of the probe head 11 may be similar to or the same as that illustrated in Figs. 4A and 4B.

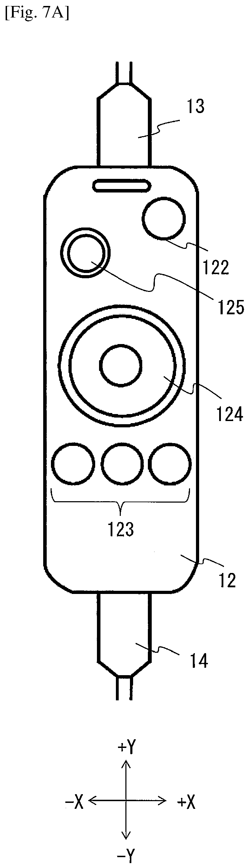

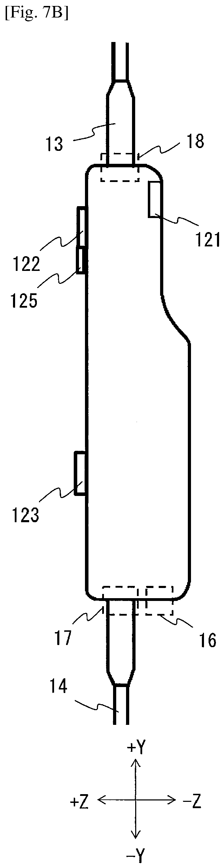

In a similar manner or the same manner, the configuration of the image capturing unit 12 is also not limited to the configuration illustrated in Figs. 3A and 3B. The image capturing unit 12 may have a configuration in which the connection place of the cables 13, 14 can be changed optionally according to a use environment. Figs. 7A and 7B illustrate a configuration in which the connection place of the cables 13, 14 can be changed optionally. In order to make it easy to understand, positions of slots 16 to 18 each representing a connection port for the cable 13 or the cable 14 are indicated by a dot line in Fig. 7B.

The image capturing unit 12 has the slots (connection ports) provided on a side in the -Y direction and a side in the +Y direction so that one of the slots (connection ports) is connected to the cable 13. That is, the image capturing unit 12 has three or more slots connectable to (detachably attached to) the cable 13 or the cable 14. For example, each of the slots may be an insertion port for a universal serial bus (USB) cable. The user attaches the cable 13 and/or the cable 14 to the image capturing unit 12 in accordance with a use condition, and places the cable 13 or the cable 14 at a most suitable position. That is, the user manually can change a connection state from the connection state in Figs. 3A and 3B to the connection state in Figs. 7A and 7B or vice versa. The connection configuration in Figs. 7A and 7B is merely an example. The cable 14 may be connected to the side facing in the +Y direction. Thus, with the provision of the three or more slots to one of which the cable 13 or the cable 14 is detachably attached, the degree of freedom for placing the cable 13 and the cable 14 is increased. Further, at least one slot 18 is provided on the side different from the side on which the other slots 16, 17 are provided, as illustrated in Figs. 7A and 7B. Accordingly, the degree of freedom for placing the cable 13 and the cable 14 is further increased. The slot 16, 17, 18 to which none of the cable 13 and the cable 14 is connected may be used for another application such as connection of a USB memory.

According to the ultrasonic probe 10 described above, the image capturing unit 12 is configured to capture an image of a nearby object, and connected to the probe head 11 through the cable 13 (first cable). Thus, the image capturing unit 12 can image the probe head 11 from a distant position. In other words, the user can move the image capturing unit 12 while holding it so that an abutment state of the probe head 11 can be properly imaged.

The image capturing unit 12 and the probe head 11 are integrated with each other through the cable 13 (first cable). Thus, the user can easily carry the ultrasonic probe 10 and the configuration of the housing can be also made compact. That is, even at a place with a large physical limitation such as an emergency ward, it is possible to perform ultrasonic measurement through the patient monitor 20 while grasping an abutment state of the probe head 11 in a simple configuration.

The image capturing unit 12 may function as a remote controller of the patient monitor 20 or the probe head 11. When the image capturing unit 12 functions as the remote controller, the user can change the setting of the patient monitor 20 or the probe head 11 on hand, while viewing the image (the ultrasonic image 21 or the photographic image 22) displayed on the patient monitor 20.

In the configuration illustrated in Fig. 2, the connector 15 is provided to be connected to the patient monitor 20. The connector 15 has a universal shape which can be inserted into a vacant slot provided in a general patient monitor 20. Therefore, as long as ultrasonic wave displaying software is installed into the patient monitor 20, the patient monitor 20 can realize an ultrasonic measurement system which can easily acquire an image equivalent to a body mark only when the ultrasonic probe 10 is connected to the patient monitor 20. A range from the connector 15 to the probe head 11 is connected by wire. Accordingly, the ultrasonic probe 10 is easily carried and high in universalness. Therefore, for example, assume that the ultrasonic wave displaying software has been installed into each of patient monitors 20 in each ward in advance. In this case, an ultrasonic image and a photographic image (equivalent to a body mark) can be checked through any of the patient monitors 20 only by detachably attaching which the ultrasonic probe 10 which is easy to carry, regardless of whether the patient monitor 20 is old or new.

On the other hand, the cable 14 through which the image capturing unit 12 and the patient monitor 20 can be connected to each other is absent from the configuration illustrated in Fig. 6. Thus, the size of the housing of the ultrasonic probe 10 can be further reduced. Thus, the ultrasonic probe 10 can have a configuration that is easier to carry, and that is further prevented from being an obstacle even at an emergency scene etc.

Description has been made on the assumption that the ultrasonic probe 10 is connected to the patient monitor 20 in the aforementioned configuration. However, the connection of the ultrasonic probe 10 is not always limited thereto. For example, the ultrasonic probe 10 may be configured to be connected to a tablet type personal computer in which ultrasonic image displaying software is installed. That is, the ultrasonic probe 10 may be electrically connected to a display device (the patient monitor 20, the tablet type personal computer, etc.) that can display an ultrasonic image. Even when the ultrasonic probe 10 is connected to the tablet type personal computer, it is possible to properly grasp an abutment state of the probe head 11 in a simple configuration as long as the ultrasonic probe 10 has the aforementioned configuration.

Next, a configuration of an ultrasonic probe 10 according to another embodiment of the presently disclosed subject matter will be described. In the following description, elements denoted by the same reference signs and names as those of the foregoing embodiment are similar to or the same as those of the foregoing embodiment unless otherwise described.

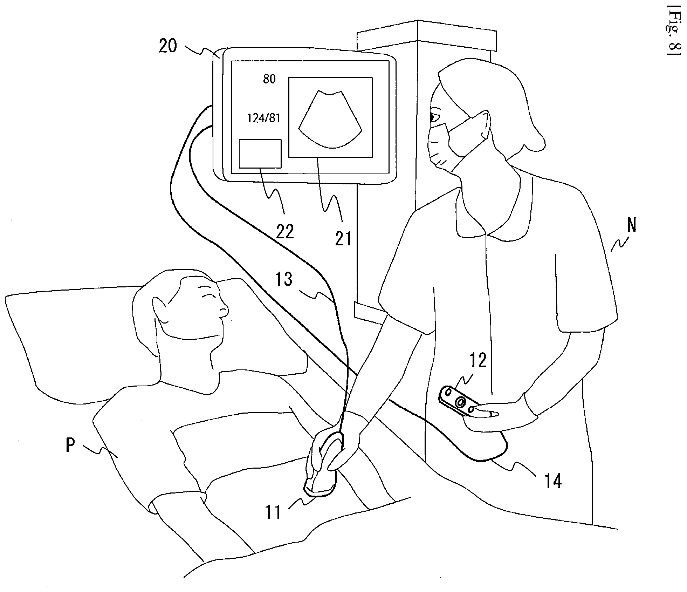

Fig. 8 is a view illustrating how the ultrasonic probe 10 may be used according to the present embodiment. A probe head 11 is connected to a patient monitor 20 through a cable 13. An image capturing unit 12 is connected to the patient monitor 20 through a cable 14. The image capturing unit 12 has an optical lens 121 that captures a photographic image of a nearby object in a similar manner to or the same manner as that of the foregoing embodiment. The image capturing unit 12 transmits the captured photographic image (an image signal or digital data showing the image) to the patient monitor 20. The image capturing unit 12 functions as a remote controller that transmits a control signal to the patient monitor 20 and the probe head 11. The image capturing unit 12 creates the control signal in accordance with operation on input interfaces (input buttons 123 etc.). The image capturing unit 12 may transmit the control signal to the probe head 11 through the cable 13 and the cable 14, or may transmit the control signal to the probe head 11 using a wireless communication function (e.g., a short range wire communication function).

Data communication between the probe head 11 and the patient monitor 20 may be also achieved by wireless. Data communication between the image capturing unit 12 and the patient monitor 20 may be also achieved by wireless. That is, as long as the ultrasonic probe 10 may have a configuration including the probe head 11 and the image capturing unit 12, housings of the probe head 11 and the image capturing unit 12 may be connected to each other by a cable or not by a cable. That is, the image capturing unit 12 may be connected to the probe head 11 by wire or by wireless.

The probe head 11 transmits an ultrasonic beam toward a body surface of a subject and receives a reflected wave from the body surface. A signal of the reflected wave acquired by the probe head 11 is supplied to the patient monitor 20 through the cable 13. Ultrasonic image data may be alternatively supplied to the patient monitor 20 after the signal of the reflected wave has been converted into the ultrasonic image data. That is, the probe head 11 may supply the image information based on the reflected wave to the patient monitor 20.

The photographic image acquired by the image capturing unit 12 and the image information acquired by the probe head 11 are supplied to the patient monitor 20. In other words, the ultrasonic probe 10 supplies the photographic image acquired by the image capturing unit 12 and the image information acquired by the probe head 11 to the patient monitor 20. The patient monitor 20 displays an ultrasonic image 21 and a photographic image 22 on a display in a similar manner to or the same manner as that according to the foregoing embodiment. While referring to the ultrasonic image 21 or the photographic image 22 displayed on the patient monitor 20, a nurse N adjusts a pointing direction (imaging direction) of the image capturing unit 12 or operates the input interfaces (the buttons etc. provided on the image capturing unit 12) to input various setting changes (setting of the probe head 11 or setting of the patient monitor 20). A control signal in accordance with operation on the input interfaces is input to the probe head 11 or the patient monitor 20.

According to the ultrasonic probe 10 described above, when the ultrasonic image is referred to, measured values or measured waveforms of vital signs are often desired to be referred to together. In addition, it is preferable that a user (medical worker) may perform grasping of an ultrasonic image of the subject, grasping of an abutment state of the probe head 11, and change of the setting of the probe head 11, etc. without changing one's gaze frequently.

The image capturing unit 12 is configured to capture a photographic image of a nearby object and to transmit a control signal to the probe head 11. That is, the image capturing unit 12 has a configuration in which both the remote controller of the probe head 11 and the imaging can be integrally performed. The image capturing unit 12 supplies the photographic image to the patient monitor 20. The probe head 11 supplies image information about an ultrasonic wave to the patient monitor 20. Therefore, the user can properly record the image equivalent to a body mark, and can control the probe head 11 while viewing the photographic image or the ultrasonic image displayed on the patient monitor 20. That is, the user can comfortably perform imaging of the body mark, the setting change (control) of the probe head 11, and checking of the ultrasonic image.

The image capturing unit 12 may be configured to transmit a control signal to the probe head 11 via the patient monitor 20. The image capturing unit 12 transmits a control signal according to operation on the input interfaces (the input buttons 123 etc.) on the image capturing unit 12 to the patient monitor 20 by wired communication or by wireless communication. The patient monitor 20 detects whether the control signal is addressed to the patient monitor 20 or addressed to the probe head 11. When the control signal indicates setting change of the probe head 11, the patient monitor 20 transmits the control signal to the probe head 11 by wired communication or by wireless communication.

Even with the configuration, the image capturing unit 12 is configured to be able to capture a photographic image of a nearby object, and can transmit the control signal to the probe head 11 and properly record the image equivalent to a body mark. The image capturing unit 12 can also control the probe head 11 in accordance with the photographic image.

While the presently disclosed subject matter has been described with reference to certain embodiments thereof, the scope of the presently disclosed subject matter is not limited to the embodiments described above, and it will be understood by those skilled in the art that various changes and modifications may be made therein as far as they fall within the scope defined by the appended claims.

## Claims

1. An ultrasonic probe (10) electrically connectable to a display device (20) configured to display an ultrasonic image (21), the ultrasonic probe (10) comprising:
an image capturing unit (12) joined to or detachably attached to a first cable (13), the image capturing unit (12) comprising a first optical lens (121) to capture an image (22) of a nearby object; and
a probe head (11) joined to or detachably attached to the first cable (13), the probe head (11) being configured to transmit an ultrasonic beam toward a body surface of a subject and to receive a reflected wave from the body surface;
**characterised in that**
the image capturing unit (12) is configured to transmit a control signal for instructing a change of settings to at least one of the display device (20) and the probe head (11) in accordance with an operation on an input interface (122, 123, 124) provided on a housing of the image capturing unit.

2. The ultrasonic probe according to claim 1, further comprising:
a connector (15) connectable to a connection port of the display device; and
a second cable (14) connected to the connector and joined to or detachably attached to the image capturing unit.

3. The ultrasonic probe according to claim 2, wherein the first cable and the second cable are arranged to extend from the image capturing unit substantially in a gravity direction when the first optical lens is oriented in a horizontal direction.

4. The ultrasonic probe according to claim 2 or 3, wherein the first cable and the second cable extend from the image capturing unit substantially in a same direction that is different from an imaging direction of the first optical lens.

5. The ultrasonic probe according to claim 2 or 3, wherein the first cable and the second cable extends from the image capturing unit such that the first cable and the second cable are arranged one behind the other when the first optical lens is viewed from the front.

6. The ultrasonic probe according to any one of claims 1 to 5, wherein the probe head comprises a marker indicating a direction in which the probe head is to be abutted on the body surface of the subject.

7. The ultrasonic probe according to any one of claims 1 to 6, wherein the image capturing unit is configured to transmit a control signal for instructing a change of settings to both the display device and the probe head in accordance with an operation on an input interface provided on a housing of the image capturing unit.

8. The ultrasonic probe according to claim 1 or 7, wherein the input interface is provided on a side opposite to a side on which the first optical lens is provided.

9. The ultrasonic probe according to claim 2, wherein the image capturing unit comprises three or more slots to which the first cable or the second cable is detachably attached.

10. The ultrasonic probe according to claim 9, wherein one of the slots is provided on a side different from a side on which another one of the slots is provided.

11. The ultrasonic probe according to any one of claims 1 to 10, wherein the display device is a patient monitor configured to display measured values and measured waveforms of vital signs.

12. An ultrasonic measurement system comprising:
a display device (20) configured to display an ultrasonic image (21); and
an ultrasonic probe (10) electrically connectable to the display device (20),
wherein the ultrasonic probe (10) comprises:
an image capturing unit (12) joined to or detachably attached to a first cable (13), the image capturing unit (12) comprising a first optical lens (121) to capture an image (22) of a nearby object; and
a probe head (11) joined to or detachably attached to the first cable (13), the probe head (11) being configured to transmit an ultrasonic beam toward a body surface of a subject and to receive a reflected wave from the body surface,
wherein the display device (20) is configured to display the ultrasonic image (21) based on the reflected wave received by the probe head (11), and a photographic image (22) captured by image capturing unit; and
**characterised in that**
the image capturing unit (12) is configured to transmit a control signal for instructing a change of settings to at least one of the display device (20) and the probe head (11) in accordance with an operation on an input interface (122, 123, 124) provided on a housing of the image capturing unit (12).

13. The ultrasonic measurement system according to claim 12, wherein the probe head comprises a marker indicating a direction in which the probe head is to be abutted on the body surface of the subject, and
the display device is configured to detect characteristics of the ultrasonic probe from at least one of a colour and a shape of the marker captured in the photographic image.

14. An ultrasonic probe comprising:
a probe head (11) configured to abut on a body surface of a subject, to transmit an ultrasonic beam toward the body surface, and to receive a reflected wave from the body surface; and
an image capturing unit (12) that is connectable to the probe head (11) by wire or by wireless to transmit a control signal to the probe head (11) or that is configured to transmit the control signal to the probe head (11) via a patient monitor (20) electrically connectable to the ultrasonic probe (10),
the image capturing unit (12) comprising a first optical lens (121) to capture an image (22) of a nearby object,
wherein the ultrasonic probe is configured to transmit a photographic image (22) acquired by the image capturing unit (12) and image information based on the reflected wave to the patient monitor (20), and
**characterised in that**
the image capturing unit (12) is configured to transmit a control signal for instructing a change of settings to at least one of the patient monitor (20) and the probe head (11) in accordance with an operation on an input interface (122, 123, 124) provided on a housing of the image capturing unit (12).

## Patentansprüche

1. Ultraschallsonde (10), die elektrisch an eine Anzeigevorrichtung (20) angeschlossen werden kann, die so ausgeführt ist, dass sie ein Ultraschallbild (21) anzeigt, wobei die Ultraschallsonde (10) umfasst:
eine Bildaufnahme-Einheit (12), die mit einem ersten Kabel (13) verbunden oder abnehmbar daran angebracht ist,
wobei die Bildaufnahme-Einheit (12) eine erste optische Linse (121) zum Aufnehmen eines Bildes (22) eines nahe gelegenen Objekts umfasst;
sowie
einen Sonden-Kopf (11), der mit dem ersten Kabel (13) verbunden oder abnehmbar daran angebracht ist, wobei der Sonden-Kopf (11) so ausgeführt ist, dass er einen Ultraschallstrahl in Richtung einer Körperoberfläche eines Patienten sendet und eine von der Körperoberfläche reflektierte Welle empfängt;
**dadurch gekennzeichnet, dass**
die Bildaufnahme-Einheit (12) so ausgeführt ist, dass sie entsprechend einer Betätigung an einer Eingabeschnittstelle (122, 123, 124), die an einem Gehäuse der Bildaufnahme-Einheit vorhanden ist, ein Steuersignal zum Anweisen einer Änderung von Einstellungen zu der Anzeigevorrichtung (20) oder/und dem Sonden-Kopf (11) sendet

2. Ultraschallsonde nach Anspruch 1, die des Weiteren umfasst:
einen Verbinder (15), der an einen Verbindungsanschluss der Anzeigevorrichtung angeschlossen werden kann; sowie
ein zweites Kabel (14), das an den Verbinder angeschlossen ist und mit der Bildaufnahme-Einheit verbunden oder abnehmbar daran angebracht ist.

3. Ultraschallsonde nach Anspruch 2, wobei das erste Kabel und das zweite Kabel so angeordnet sind, dass sie sich von der Bildaufnahme-Einheit im Wesentlichen in einer Schwerkraftrichtung erstreckt, wenn die erste optische Linse in einer horizontalen Richtung ausgerichtet ist.

4. Ultraschallsonde nach Anspruch 2 oder 3, wobei das erste Kabel und das zweite Kabel so angeordnet sind, dass sie sich von der Bildaufnahme-Einheit im Wesentlichen in ein und derselben Richtung erstrecken, die sich von einer Bilderzeugungs-Richtung der ersten optischen Linse unterscheidet.

5. Ultraschallsonde nach Anspruch 2 oder 3, wobei sich das erste Kabel und das zweite Kabel so von der Bildaufnahme-Einheit aus erstrecken, dass das erste Kabel und das zweite Kabel, bei Sicht auf die erste optische Linse von vorn, hintereinander angeordnet sind.

6. Ultraschallsonde nach einem der Ansprüche 1 bis 5, wobei der Sonden-Kopf eine Markierung umfasst, die eine Richtung angibt, in der der Sonden-Kopf an der Körperoberfläche des Patienten anliegen soll.

7. Ultraschallsonde nach einem der Ansprüche 1 bis 6, wobei die Bildaufnahme-Einheit so ausgeführt ist, dass sie entsprechend einer Betätigung an einer Eingabeschnittstelle, die an einem Gehäuse der Bildaufnahme-Einheit vorhanden ist, ein Steuersignal zum Anweisen einer Änderung von Einstellungen sowohl zu der Anzeigevorrichtung als auch zu dem Sonden-Kopf sendet.

8. Ultraschallsonde nach Anspruch 1 oder 7, wobei die Eingabeschnittstelle an einer Seite vorhanden ist, die einer Seite gegenüberliegt, an der die erste optische Linse vorhanden ist.

9. Ultraschallsonde nach Anspruch 2, wobei die Bildaufnahme-Einheit drei oder mehr Anschlüsse aufweist, an denen das erste Kabel oder das zweite Kabel abnehmbar angebracht ist.

10. Ultraschallsonde nach Anspruch 9, wobei einer der Anschlüsse an einer Seite vorhanden ist, die sich von einer Seite unterscheidet, an der ein anderer der Anschlüsse vorhanden ist.

11. Ultraschallsonde nach einem der Ansprüche 1 bis 10, wobei die Anzeigevorrichtung ein Patientenmonitor ist, der so ausgeführt ist, dass er Messwerte und gemessene Wellenformen von Vitalzeichen anzeigt.

12. Ultraschall-Messsystem, das umfasst:
eine Anzeigevorrichtung (20), die so ausgeführt ist, dass sie ein Ultraschallbild (21) anzeigt; sowie
eine Ultraschallsonde (10), die elektrisch an die Anzeigevorrichtung (20) angeschlossen werden kann,
wobei die Ultraschallsonde (10) umfasst:
eine Bildaufnahme-Einheit (12), die mit einem ersten Kabel (13) verbunden oder abnehmbar daran angebracht ist, wobei die Bildaufnahme-Einheit (12) eine erste optische Linse (121) zum Aufnehmen eines Bildes (22) eines nahe gelegenen Objekts umfasst; sowie einen Sonden-Kopf (11), der mit dem ersten Kabel (13) verbunden oder abnehmbar daran angebracht ist, wobei der Sonden-Kopf (11) so ausgeführt ist, dass er einen Ultraschallstrahl in Richtung einer Körperoberfläche eines Patienten sendet und eine von der Körperoberfläche reflektierte Welle empfängt,
wobei die Anzeigevorrichtung (20) so ausgeführt ist, dass sie das Ultraschallbild (21) auf Basis der durch den Sonden-Kopf (11) empfangenen reflektierten Welle sowie ein mit der Bildaufnahme-Einheit aufgenommenes fotografisches Bild (22) anzeigt; und
**dadurch gekennzeichnet, dass**
die Bildaufnahme-Einheit (12) so ausgeführt ist, dass sie entsprechend einer Betätigung an einer Eingabeschnittstelle (122, 123, 124), die an einem Gehäuse der Bildaufnahme-Einheit (12) vorhanden ist, ein Steuersignal zum Anweisen einer Änderung von Einstellungen zu der Anzeigevorrichtung (20) oder/und dem Sonden-Kopf (11) sendet.

13. Ultraschall-Messsystem nach Anspruch 12, wobei der Sonden-Kopf eine Markierung umfasst, die eine Richtung angibt, in der der Sonden-Kopf an der Körperoberfläche des Patienten anliegen soll, und
die Anzeigevorrichtung so ausgeführt ist, dass sie Eigenschaften der Ultraschallsonde anhand einer Farbe oder/und einer Form der in dem fotografischen Bild aufgenommenen Markierung erfasst.

14. Ultraschallsonde, die umfasst:
einen Sonden-Kopf (11), der so ausgeführt ist, dass er an einer Körperoberfläche eines Patienten anliegt, löschen einen Ultraschallstrahl in Richtung der Körperoberfläche sendet und eine von der Körperoberfläche reflektierte Welle empfängt; sowie
eine Bildaufnahme-Einheit (12), die mittels Kabel oder drahtlos mit dem Sonden-Kopf (11) verbunden werden kann,
um ein Steuersignal zu dem Sonden-Kopf (11) zu senden, oder die so ausgeführt ist,
dass sie das Steuersignal zu dem Sonden-Kopf (11) über einen Patientenmonitor (20) sendet, der elektrisch an die Ultraschallsonde (10) angeschlossen werden kann,
wobei die Bildaufnahme-Einheit (12) eine erste optische Linse (121) zum Aufnehmen eines Bildes (22) eines nahe gelegenen Objektes umfasst;
die Ultraschallsonde so ausgeführt ist, dass sie ein von der Bildaufnahme-Einheit (12) erfasstes fotografisches Bild (22) sowie Bildinformationen auf Basis der reflektierten Welle zu dem Patientenmonitor (20) sendet, und
**dadurch gekennzeichnet, dass**
die Bildaufnahme-Einheit (12) so ausgeführt ist, dass sie entsprechend einer Betätigung an einer Eingabeschnittstelle (122, 123, 124), die an einem Gehäuse der Bildaufnahme-Einheit (12) vorhanden ist, ein Steuersignal zum Anweisen einer Änderung von Einstellungen zu dem Patientenmonitor (20) oder/und dem Sonden-Kopf (11) sendet.

## Revendications

1. Sonde à ultrasons (10) raccordable électriquement à un dispositif d'affichage (20) configuré pour afficher une image ultrasonique (21), la sonde à ultrasons (10) comprenant :
une unité de capture d'image (12) jointe à ou rattachée de manière amovible à un premier câble (13), l'unité de capture d'image (12) comprenant une première lentille optique (121) pour capturer une image (22) d'un objet proche ; et
une tête de sonde (11) jointe au ou rattachée de manière amovible au premier câble (13), la tête de sonde (11) étant configurée pour transmettre un faisceau ultrasonique vers une surface corporelle d'un sujet et pour recevoir une onde réfléchie provenant de la surface corporelle ;
**caractérisée en ce que**
l'unité de capture d'image (12) est configurée pour transmettre un signal de commande pour signifier un changement de réglages à au moins un du dispositif d'affichage (20) et de la tête de sonde (11) conformément à une opération sur une interface d'entrée (122, 123, 124) disposée sur un boîtier de l'unité de capture d'image.

2. La sonde à ultrasons selon la revendication 1, comprenant en outre :
un connecteur (15) raccordable à un port de connexion du dispositif d'affichage ; et
un deuxième câble (14) connecté au connecteur et joint à ou rattaché de manière amovible à l'unité de capture d'image.

3. La sonde à ultrasons selon la revendication 2, dans laquelle le premier câble et le deuxième câble sont disposés pour s'étendre depuis l'unité de capture d'image substantiellement dans une direction de gravité lorsque la première lentille optique est orientée dans une direction horizontale.

4. La sonde à ultrasons selon la revendication 2 ou 3, dans laquelle le premier câble et le deuxième câble s'étendent depuis l'unité de capture d'image substantiellement dans une même direction qui est différente d'une direction d'imagerie de la première lentille optique.

5. La sonde à ultrasons selon la revendication 2 ou 3, dans laquelle le premier câble et le deuxième câble s'étendent depuis l'unité de capture d'image de sorte que le premier câble et le deuxième câble soient disposés l'un derrière l'autre lorsque la première lentille optique est vue depuis l'avant.

6. La sonde à ultrasons selon l'une quelconque des revendications 1 à 5, dans laquelle la tête de sonde comprend un marqueur indiquant une direction dans laquelle la tête de sonde doit s'appuyer sur la surface corporelle du sujet.

7. La sonde à ultrasons selon l'une quelconque des revendications 1 à 6, dans laquelle l'unité de capture d'image est configurée pour transmettre un signal de commande pour signifier un changement de réglages à la fois au dispositif d'affichage et à la tête de sonde conformément à une opération sur une interface d'entrée disposée sur un boîtier de l'unité de capture d'image.

8. La sonde à ultrasons selon la revendication 1 ou 7, dans laquelle l'interface d'entrée est disposée sur un côté opposé à un côté sur lequel la première lentille optique est disposée.

9. La sonde à ultrasons selon la revendication 2, dans laquelle l'unité de capture d'image comprend trois connecteurs ou plus auxquels le premier câble ou le deuxième câble est rattaché de manière amovible.

10. La sonde à ultrasons selon la revendication 9, dans laquelle un des connecteurs est disposé sur un côté différent d'un côté sur lequel un autre des connecteurs est disposé.

11. La sonde à ultrasons selon l'une quelconque des revendications 1 à 10, dans laquelle le dispositif d'affichage est un moniteur de patient configuré pour afficher des valeurs mesurées et des formes d'onde mesurées de signes vitaux.

12. Système de mesure à ultrasons comprenant :
un dispositif d'affichage (20) configuré pour afficher une image ultrasonique (21) ; et
une sonde à ultrasons (10) raccordable électriquement au dispositif d'affichage (20),
dans lequel la sonde à ultrasons (10) comprend :
une unité de capture d'image (12) jointe à ou rattachée de manière amovible à un premier câble (13), l'unité de capture d'image (12) comprenant une première lentille optique (121) pour capturer une image (22) d'un objet proche ; et
une tête de sonde (11) jointe au ou rattachée de manière amovible au premier câble (13), la tête de sonde (11) étant configurée pour transmettre un faisceau ultrasonique vers une surface corporelle d'un sujet et pour recevoir une onde réfléchie provenant de la surface corporelle,
dans lequel le dispositif d'affichage (20) est configuré pour afficher l'image ultrasonique (21) selon l'onde réfléchie reçue par la tête (11), et une image photographique (22) capturée par l'unité de capture d'image ; et
**caractérisé en ce que**
l'unité de capture d'image (12) est configurée pour transmettre un signal de commande pour signifier un changement de réglages à au moins un du dispositif d'affichage (20) et de la tête de sonde (11) conformément à une opération sur une interface d'entrée (122, 123, 124) disposée sur un boîtier de l'unité de capture d'image (12).

13. Le système de mesure à ultrasons selon la revendication 12, dans lequel la tête de sonde comprend un marqueur indiquant une direction dans laquelle la tête de sonde doit s'appuyer sur la surface corporelle du sujet, et
le dispositif d'affichage est configuré pour détecter des caractéristiques de la sonde à ultrasons à partir d'au moins une d'une couleur et d'une forme du marqueur capturé dans l'image photographique.

14. Sonde à ultrasons comprenant :
une tête de sonde (11) configurée pour s'appuyer sur une surface corporelle d'un sujet, pour transmettre un faisceau ultrasonique vers la surface corporelle, et pour recevoir une onde réfléchie provenant de la surface corporelle ; et
une unité de capture d'image (12) qui est raccordable à la tête de sonde (11) par câble ou par liaison sans fil pour transmettre un signal de commande à la tête de sonde (11) ou qui est configuré pour transmettre le signal de commande à la tête de sonde (11) via un moniteur de patient (20) raccordable électriquement à la sonde à ultrasons (10),
l'unité de capture d'image (12) comprenant une première lentille optique (121) pour capturer une image (22) d'un objet proche,
dans laquelle la sonde à ultrasons est configurée pour transmettre une image photographique (22) acquise par l'unité de capture d'image (12) et des informations d'image reposant sur l'onde réfléchie au moniteur de patient (20), et
**caractérisée en ce que**
l'unité de capture d'image (12) est configurée pour transmettre un signal de commande pour signifier un changement de réglages à au moins un du moniteur de patient (20) et de la tête de sonde (11) conformément à une opération sur une interface d'entrée (122, 123, 124) disposée sur un boîtier de l'unité de capture d'image (12) .
